# EUROPEAN PATENT SPECIFICATION

(11) **EP 3 964 289 B1**
(45) Date of publication and mention of the grant of the patent: **20.09.2023**
(21) Application number: 21205180.9
(22) Date of filing: 05.01.2018
(51) Int. Cl.: B01L 1/02, B25J 21/02, C12M 1/12, G21F 7/04

(54) **ISOLATOR SYSTEM**
ISOLATORSYSTEM
SYSTÈME D'ISOLATEUR

(43) Date of publication of application: 09.03.2022
(62) Divisional of application: 18898181.5
(73) Proprietor: JGC Japan Corporation, Yokohama-shi, Kanagawa 220-6001 (JP)
(72) Inventor: KOJIMA, Takeshi, Yokohama-shi, 220-6001 (JP)
(74) Representative: TBK

(56) References cited:
- WO-A1-2004/025667
- WO-A1-2016/183513
- JP-A- 2015 226 970
- US-A- 5 730 777

## Description

### Technical Field

The present invention relates to a technique of exhausting a gas in a glove box which is provided in an isolator system.

### Background Art

There is known a glove box (hereinafter, which is also referred to as a "GB") that is capable of performing the handling of a substance in an operation space which is isolated from outside (internal operation), in order to prevent the substance from leaking outside when handling the substance affecting the human body or the environment.

For example, a glove which is inserted into the operation space is connected to a front surface of the GB, and an operator inserts the arm into the glove to perform an internal operation in the operation space.

At the time, in order to prevent the leak of a substance being handled, the operation space (the inside of the GB) is maintained at a pressure (negative pressure) which is lower than an outside pressure.

As a method for maintaining the inside of the GB at a negative pressure, Patent Documents 1 and 2 describe a technique of controlling forced exhaust means (Patent Document 1) for forcibly exhausting a gas in the GB or an exhaust valve (Patent Document 2) provided in an exhaust system, based on a result of detecting a differential pressure between inside and outside the GB.
A conventional isolator system according to the preamble portion of claim 1 of the present application is disclosed in Patent Document 3. A further conventional isolator system is disclosed in Patent Document 4.

### Citation List

### Patent Document

Patent Document 1: JP-A-2013-86183
Patent Document 2: Japanese Patent No. 2,537,689
Patent Document 3: US Patent Publication No. US 5 730 777 A
Patent Document 4: Japanese Patent Publication No. JP 2015 226970 A

### Summary of the Invention

### Technical Problem

As described above, generally, a method for controlling the exhaust of a gas from the GB based on the result of detecting the differential pressure between inside and outside the GB is used as the method for maintaining the inside of the GB at a negative pressure.

However, in order to realize this control based on the detection of the differential pressure, it is necessary to detect the differential pressure between inside and outside the GB with a high sensitivity, and provide a plurality of pressure regulation valves with good responsiveness (Patent Document 1: pressure sensors 41 and 42, a first opening and closing valve 13, and a second opening and closing valve 22, and Patent Document 2: a differential pressure gauge 16, a negative pressure gauge 18, a gas supply valve 22, an exhaust valve 26, and a bypass valve 32) . For this reason, a pressure control mechanism of the GB becomes large in scale, and the above-described requirement also becomes a factor which increases the cost of the apparatus.

In addition, in the above-described control method, in a case where a large damage opening is formed in the GB due to the falling off of the glove or the like, since the pressure difference between inside and outside the GB decreases rapidly, control is performed to greatly increase the amount of exhaust from the GB within a short period of time in order to secure a target differential pressure.

As a result, a pressure change occurs, so that the inside of an operation room where the GB is arranged enters a rapidly reduced pressure atmosphere, and it is not possible to properly maintain the differential pressure between rooms with respect to an area adjacent to the operation room, which is a concern.

The invention is made under the above-described background, and provides an isolator system that is capable of stably maintaining a pressure in an operation space at a negative pressure with a simple configuration.

### Solution to Problem

The present invention solves the problem by an isolator system according to claim 1. An advantageous further development is subject-matter of the dependent claim.

### Advantageous Effects of the Invention

According to the invention, since an exhaust flow rate of a gas from an exhaust port of a glove box is regulated to an exhaust flow rate where a differential pressure which is set in advance can be formed in a differential pressure forming portion provided in an intake port of the glove box, with a simple configuration, it is possible to maintain the internal pressure of the glove box at a negative pressure.

### Brief Description of the Drawings

Fig. 1 is a configuration diagram of an example of an isolator system, which is not in accordance with the invention.
Fig. 2 is a diagram of the operation of the GB when a glove falls off.
Fig. 3 is a configuration diagram of a GB where a differential pressure regulation valve is provided on an intake port side.
Fig. 4 is a configuration diagram of an isolator system which exhausts a gas in an operation room.
Fig. 5 is a configuration diagram of an isolator system according to an embodiment of the present invention.
Fig. 6 is a diagram of the operation of a GB according to the embodiment when a glove falls off.
Fig. 7 is a configuration diagram of a GB which is configured to be detachable from a plurality of reaction vessels.
Fig. 8 is a diagram showing the GB before being connected to the reaction vessel.
Fig. 9 is a diagram showing the GB after being connected to the reaction vessel.

### Description of Embodiment

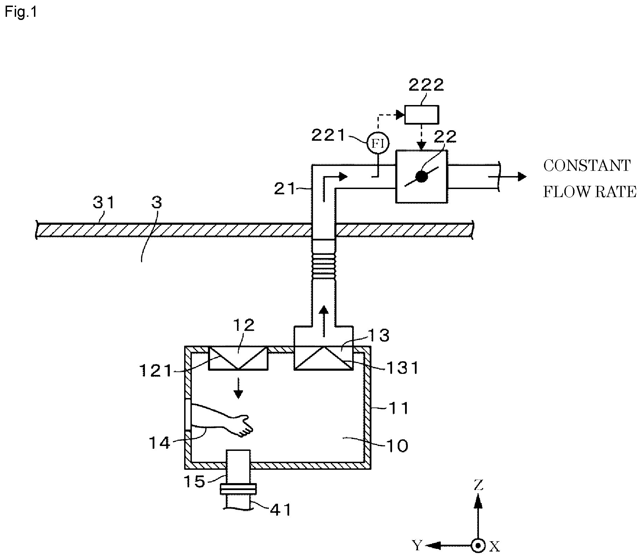

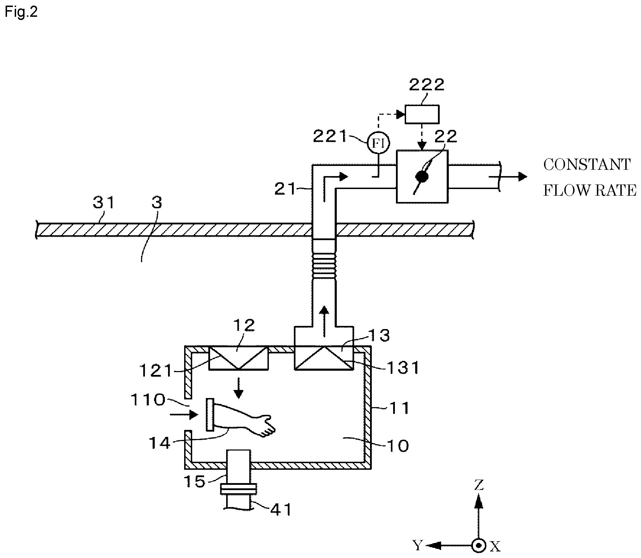

Hereinafter, a configuration example of an isolator system including a glove box (GB) 11 for inputting a raw material into a reaction vessel 4 which proceeds with a reaction for producing, for example, an intermediate product or a final product will be described with reference to Figs. 1 and 2. The supply amount of a gas (for example, air) from outside to an operation room 3 provided with the reaction vessel 4 is restricted for the purpose of maintaining a differential pressure between the operation room 3 and an adjacent area, or the like.

The GB 11 has, for example, a structure where a panel portion (not illustrated) made of glass is provided in a front surface of a housing made of metal, and the GB 11 forms an operation space 10 which is isolated from an outside space.

A glove 14 is airtightly attached to the front surface of the GB 11 which is arranged to face an operator. The glove 14 corresponds to an operation portion for performing an internal operation in the operation space 10.

In addition to the glove 14, as another example of the operation portion provided in the GB 11, a side surface of the operation space 10 may include, for example, a bag import to which a carry in and carry out bag for carrying in and out a substance from the GB 11 is attached (the carry in and carry out bag and the bag import are not illustrated).

A bottom surface of the GB 11 is provided with an input pipe 15 which is configured to be connectable to a receiving pipe 41 provided in the reaction vessel 4, and through which a raw material is input thereinto. The input pipe 15 is provided to penetrate a bottom plate of the GB 11. A lower end portion of the input pipe 15 is provided with a flange which can be fastened to the receiving pipe 41. On the other hand, an upper end portion of the input pipe 15 is inserted into the operation space 10.

For example, an upper surface of the GB 11 is provided with an intake port 12 for taking a gas (for example, air) in the operation room 3 into the operation space 10, and an exhaust port 13 for exhausting a gas (for example, air) in the operation space 10.

The intake port 12 is provided with a filter 121 which forms a differential pressure forming portion in thisexample. The differential pressure forming portion (the filter 121) has the function of forming a differential pressure which is set in advance between the inside (the operation space 10) and the outside (the operation room 3) of the GB 11.

As the filter 121 provided in the intake port 12, it is possible to select a particle filter such as a high efficiency particulate air (HEPA) filter or a mesh filter with coarser meshes in order to answer the purpose such as preventing dust or foreign matter from infiltrating into the operation space 10 in addition to the function as the above-described differential pressure forming portion.

In addition, the exhaust port 13 is provided with a filter 131 for preventing the leak of a substance being handled (in this example, a raw material for producing an intermediate product or a final product) in the operation space 10. An exhaust pipe 21 is connected to an outlet side of the exhaust port 13.

For example, the exhaust pipe 21 penetrates a ceiling portion 31 of the operation room 3 to extend outside the operation room 3 and be connected to an exhaust mechanism (not illustrated) including an exhaust fan and the like.

The exhaust pipe 21 which leads out to an upper surface side of the operation room 3 is provided with a flow rate regulation mechanism 22 for regulating the exhaust flow rate of the gas which is exhausted from the exhaust port 13 to the exhaust pipe 21. The flow rate regulation mechanism 22 includes a damper capable of regulating the opening angle and the like. A controller 222 regulates the opening angle based on a result of measuring the exhaust flow rate with a flow meter 221 provided in the exhaust pipe 21, such that an actual exhaust flow rate approaches a target exhaust flow rate (target flow rate) which is set in advance.

In the isolator system with the above-described configuration, the target flow rate for the exhaust flow rate of the gas which is regulated by the flow rate regulation mechanism 22 is set from the following three viewpoints.

From a first viewpoint, the target flow rate is set such that the differential pressure which is set in advance is formed in the filter 121 which is the differential pressure forming portion provided in the intake port 12.

When a pressure change along the flow of the gas which is taken into the GB 11 (the operation space 10) and then is exhausted to the exhaust pipe 21 through the exhaust port 13 is observed, the pressure of the gas becomes the highest in the operation room 3, and becomes the lowest in an inflow portion of the exhaust mechanism (not illustrated) which is positioned at a downstream end of the exhaust pipe 21.

At the time, in a case where the intake port 12 is provided with the filter 121 which is the differential pressure forming portion, the differential pressure is formed in the filter 121; and thereby, it is possible to maintain a negative pressure state where the pressure in the operation space 10 is lower than the pressure in the operation room 3.

Therefore, based on a set pressure value which has to be maintained in the operation space 10 while an internal operation is performed or the like, the exhaust flow rate where a differential pressure which corresponds to the pressure difference between the pressure of the outside (the operation room 3) of the GB 11 and the pressure in the operation space 10 can be formed in the filter 121 is set as the target flow rate.

As an example, it is possible to provide a case where the target flow rate is set such that a differential pressure of 100 Pa (initial value before the differential pressure increases due to the collection of particles or the like) within a range of 50 to 200 Pa is formed as an example of the differential pressure which is formed in the filter 121. Incidentally, since the target flow rate is changed depending on the volume of the operation space 10, the opening area of the intake port 12, and the like, it is difficult to generally specify the target flow rate.

From a second viewpoint, the target flow rate is set such that when the GB 11 is damaged, the leak of a substance being handled can be prevented.

For example, in a case where the GB 11 is a housing made of metal, there is a possibility that the glove 14 attached to the front surface of the GB 11 falls off from an attachment port thereof, or the carry in and carry out bag is removed from the bag import provided in a side surface of the GB 11, and thus, a relatively large opening (damage opening 110) is formed.

In a case where the damage opening 110 is formed, there is almost no differential pressure between the operation room 3 and inside of the operation space 10, and for example, the flow of a fine-particle substance being handled, or the flow of a gas generated from the substance being handled, both of which flow from the inside of the operation space 10 to the operation room 3, is formed, which is also a concern.

Therefore, the target flow rate may be set such that even in a case where the damage opening 110 described above is formed, an airflow which flows from the operation room 3 into the operation space 10 at a predetermined lowest air speed or greater is formed by continuously exhausting the gas inside the damage opening 110 through the exhaust port 13.

As an example of the lowest air speed of the gas which flows in through the damage opening 110, it is possible to provide an air speed of 0.5 m/s within a range of 0.4 to 1.0 m/s. It is possible to specify the target flow rate by multiplying the lowest air speed by the opening area of the damage opening 110 (the attachment port for the glove 14 or the bag import).

Incidentally, in a case where the exhaust flow rate is set based on assuming the event where the damage opening 110 which is the largest may occur such as the falling off of the glove 14 or the bag import, even in a case where the event occurs such as the occurrence of the damage opening 110 which is smaller, the flow speed of the gas flowing into the damage opening 110 can be maintained at the predetermined lowest air speed or greater.

A third viewpoint is that a pressure increase in the operation space 10 which is involved by performing an internal operation has to be prevented. The internal operation in the GB 11, for example, the case of inserting the glove 14 which leads outside into the operation space 10 through the attachment port, the case of raising a cylinder-shaped elevation mechanism (not illustrated) which is provided in the operation space 10, or the like, involves a change in the internal volume of the operation space 10.

At the time, in a case where the change in the internal volume occurs at a speed exceeding the exhaust flow rate in the operation space 10, the pressure in the operation space 10 increases to become a positive pressure, which is a concern.

Therefore, the internal operation which involves a change in the internal volume of the operation space 10 is assumed, and an exhaust flow rate larger than the change speed of the internal volume is set as the target flow rate; and thereby, it is possible to prevent a pressure increase in the operation space 10 caused by an internal operation.

In a case where the target flow rates which are obtained from the above-described three viewpoints differ from each other, the largest target flow rate is adopted and the exhaust flow rate is regulated; and thereby, it is possible to attain the effects such as maintaining a negative pressure in the operation space 10, preventing the outflow of the gas when the damage opening 110 is formed, and maintaining a negative pressure in the operation space 10 when an internal operation which involves a change in the internal volume is performed.

Incidentally, in the GB 11, it is not an indispensable requirement that the target flow rate is set in order to satisfy all of the viewpoints. The target flow rate may be set in order to satisfy at least the first viewpoint (forming the differential pressure for maintaining a negative pressure in the operation space 10) depending on the function required for the GB 11.

The operation of the isolator system with the above-described configuration will be described.

In performing an internal operation, while the exhaust flow rate is regulated by the flow rate regulation mechanism 22, the input pipe 15 is connected to the receiving pipe 41 of the reaction vessel 4 into which a raw material is input or the like, and thus, the operation space 10 which is isolated from outside is formed (Fig. 1) . At the time, in a case where the gas enters and exits from the reaction vessel 4 through the receiving pipe 41, the exhaust air volume may be regulated in response to the entering and exiting of the gas. As the operation space 10 is formed, the gas in the operation room 3 is taken into the operation space 10 through the intake port 12, and the differential pressure which is set in advance is formed in the filter 121, and thus, the inside of the operation space 10 is maintained in a negative pressure state with respect to the operation room 3.

Thereafter, an operator performs a necessary internal operation using the operation portion such as the glove 14 or the bag import, and in addition thereto, instruments and the like provided in the operation space 10.

At the time, even in a case where the internal operation involves a change in the internal volume of the operation space 10, the exhaust flow rate of the gas from the exhaust port 13 is set to an exhaust flow rate which is obtained by adding an exhaust flow rate, which is equivalent to the change speed of the internal volume when the internal volume decreases, to the already described exhaust flow rate for maintaining a negative pressure in the operation space 10; and thereby, it is possible to maintain a negative pressure state in the operation space 10.

Here, the exhaust flow rate is, for example, an exhaust flow rate where when the pressure of a secondary side of the flow rate regulation mechanism 22 is maintained constant, the pressure difference between the operation room 3 and the secondary side of the flow rate regulation mechanism 22 and a pressure loss which occurs in the filter 121 or 131, the exhaust pipe 21, or the flow rate regulation mechanism 22 balance out each other.

Subsequently, as illustrated in Fig. 2, it is assumed that as the operation portion such as the glove 14 falls off, the damage opening 110 is formed. In this case, the gas flows in through the damage opening 110 more easily than through the intake port 12 where a large differential pressure is formed, the pressure in the operation space 10 increases, and almost no pressure loss occurs in the filter 121. For this reason, in a case where any regulation is not performed, the exhaust flow rate increases such that the pressure loss in the filter 121 before the damage opening 110 is formed and the pressure loss occurring in the filter 131, the exhaust pipe 21, or the flow rate regulation mechanism 22 balance out each other.

However, in the isolator system of this example, the flow rate regulation mechanism 22 prevents an increase in the exhaust flow rate which is involved by a pressure increase in the operation space 10, and performs the regulation of the flow rate in order to maintain the exhaust flow rate constant.

As a result, the flow speed of the gas which flows in through the damage opening 110 can be maintained at the predetermined lowest air speed (for example, 0.5 m/s), and the outflow of a substance which is being handled in the operation space 10 can be prevented.

Then, at the time, in the GB 11 of this example, since the exhaust flow rate from the exhaust port 13 is maintained constant before and after the damage opening 110 is formed, a large pressure change does not occur in the operation room 3.

The isolator system according to this example provides the following effects. Since the exhaust flow rate of the gas from the exhaust port 13 of the GB 11 is regulated to the exhaust flow rate where the differential pressure which is set in advance can be formed in the differential pressure forming portion (the filter 121) provided in the intake port 12 of the GB 11, with a simple configuration, the internal pressure of the GB 11 can be maintained at a negative pressure.

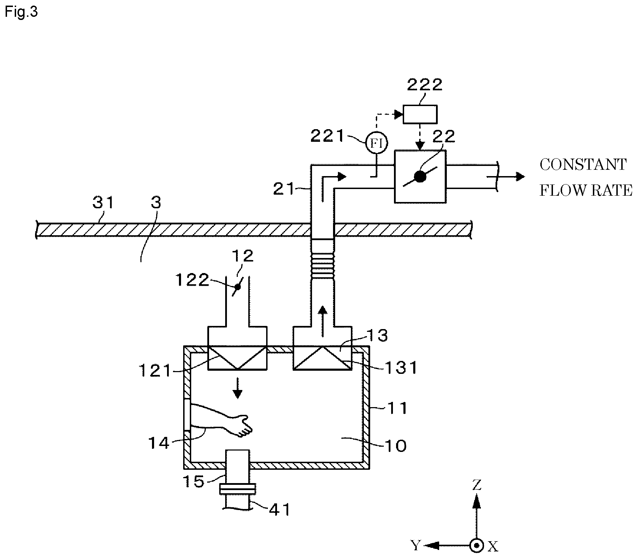

Here, the invention is not limited to a case where the differential pressure forming portion provided in the intake port 12 includes only the filter 121 which is described with reference to Figs. 1 and 2. For example, as illustrated in Fig. 3, in addition to the filter 121, a differential pressure regulation valve 122 which is a damper or the like may be provided. In addition, the configuration where the installation of the filter 121 is omitted and only the differential pressure regulation valve 122 is provided in the intake port 12 may be adopted (not illustrated).

In addition, as illustrated in Figs. 1 and 2, in a case where the flow rate regulation mechanism 22 is provided in the exhaust pipe 21 which leads out outside the operation room 3, since a maintenance worker completes maintenance without entering the operation room 3, the maintenance of the flow rate regulation mechanism 22 is facilitated.

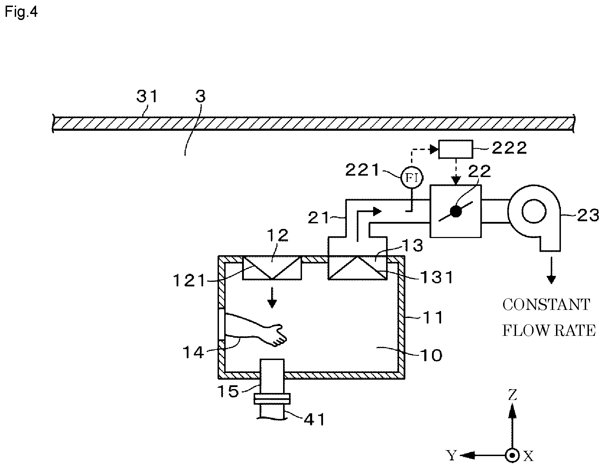

However, it is not an indispensable requirement that the exhaust pipe 21 extends outside the operation room 3. For example, as illustrated in Fig. 4, the configuration where the exhaust pipe 21 is connected to an exhaust fan 23 provided in the operation room 3, and the gas exhausted from the exhaust port 13 returns to the operation room 3 may be adopted.

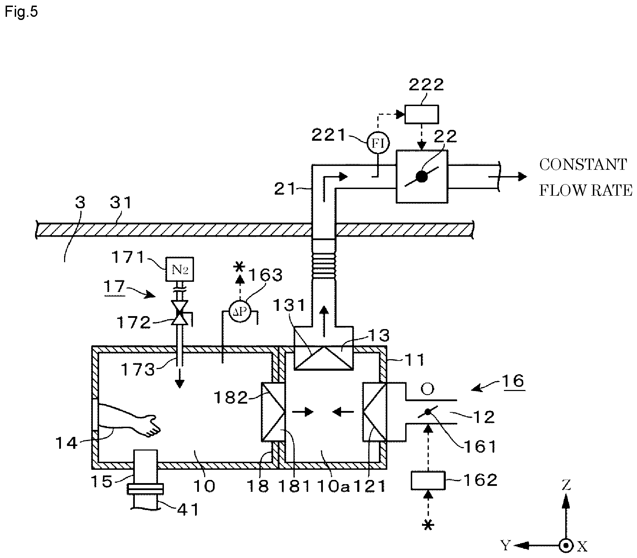

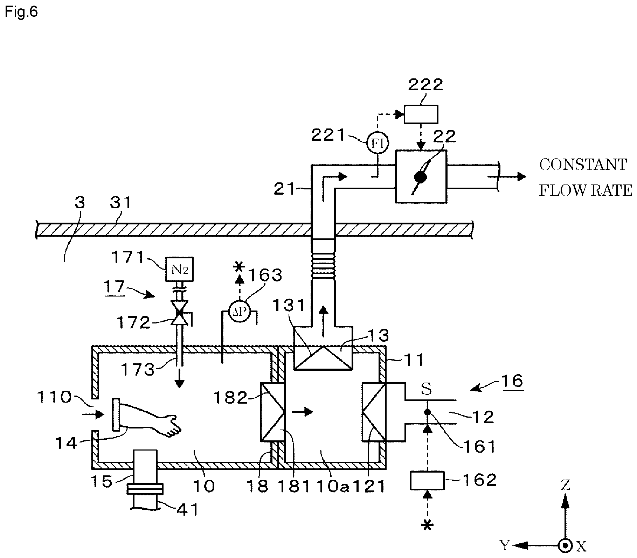

Subsequently, an isolator system according to an embodiment of the present invention will be described with reference to Figs. 5 and 6. In each drawing which will be described hereinafter, the reference signs which are common to Figs. 1 to 4 are assigned to the configuration elements which are common to the GB 11 described with reference to these drawings.

The GB 11 of this example is applicable to a case where in maintaining an atmosphere of an inert gas such as a nitrogen gas in the operation space 10, the amount of consumption of the inert gas is reduced, or the like.

The inside of the GB 11 is partitioned into the operation space 10 and a buffer space 10a by a partition member 18, and the operation space 10 and the buffer space 10a communicate with each other through a communication port 181. In this example, the partition member 18 is provided with a filter 182, and a substance which is being handled in the operation space 10 is not allowed to enter the buffer space 10a.

The partition member 18 is provided with the glove 14 or the input pipe 15 which is already described. Instead of the intake port 12, an operation space side gas supply unit 17 for supplying an inert gas (in this example, a nitrogen gas), which is an operation space gas, to the operation space 10 is provided in the partition member 18.

For example, the operation space side gas supply unit 17 includes a nitrogen gas supply unit 171 including a nitrogen storage unit or a flow rate regulation unit, and a nitrogen gas supply pipe 173 which is provided with an opening and closing valve 172. A distal end portion of the nitrogen gas supply pipe 173 is inserted into the operation space 10.

Then, in the GB 11 of this example, the intake port 12, which is provided with, for example, the filter 121 that is the differential pressure forming portion, is provided in the buffer space 10a. Furthermore, a supply stop mechanism 161 which is an opening and closing damper or the like is provided in the intake port 12, and is capable of stopping taking the gas in through the intake port 12.

The intake port 12 and the supply stop mechanism 161 are equivalent to a buffer side gas supply unit 16 which supplies a buffer gas (in this example, air) to the buffer space 10a.

The supply stop mechanism 161 is instructed by a controller 162 to perform an opening and closing operation based on a result of measuring a differential pressure between inside and outside the operation space 10 with a differential pressure measurement unit 163. The controller 162 controls the operation of the supply stop mechanism 161 such that when the differential pressure in the operation space 10 with respect to the operation room 3 reaches a negative pressure with a magnitude set in advance, the supply stop mechanism 161 is closed to stop the gas (buffer gas) from being taken into the buffer space 10a.

Incidentally, the controller 162 does not necessarily have an opening angle regulation function. In addition, the taking in of the buffer gas may be stopped using a manual damper which regulates the pressure, and a lid where only closing is allowed. On the contrary, the configuration where the opening angle of a damper is manually regulated and only the closing operation of the damper is automatically performed may be adopted.

Here, similar to the GB 11 according to the first example which is described with reference to Figs. 1 and 2, the target flow rate for the exhaust flow rate from the exhaust port 13 is set from, for example, the above-described three viewpoints (forming a negative pressure in the operation space 10, ensuring the flow speed of the gas which flows into the operation space 10 when the damage opening 110 is formed, and maintaining a negative pressure in the operation space 10 when an internal operation which involves a change in internal volume is performed).

Furthermore, in the GB 11 of this example, the flow rate of the operation space gas (nitrogen gas) supplied from the operation space side gas supply unit 17 is set at a flow rate where a pressure loss occurring when the buffer gas (air) taken in from the intake port 12 passes through the filter (differential pressure forming portion) 121 provided in the buffer side gas supply unit 16 becomes larger than a pressure loss occurring when the operation space gas passes through the filter 182 provided in the partition member 18.

The pressure in the operation space 10 and the buffer space 10a can be maintained at a negative pressure with respect to the operation room 3 by the differential pressure which occurs in the filter 121 of the intake port 12 by the above-described setting of the flow rate. Then, in a case where the supply amount of the operation space gas which is an inert gas is restricted to a small amount, the pressure in the operation space 10 approaches the pressure of the buffer space 10a; and thereby, it is possible to maintain a negative pressure in the operation space 10 while filling the operation space 10 with the inert gas.

Subsequently, an operation of an isolator system according to the embodiment will be described.

In performing an internal operation, while the exhaust flow rate is regulated by the flow rate regulation mechanism 22, the input pipe 15 is connected to the receiving pipe 41 of the reaction vessel 4, and thus, the operation space 10 which is isolated from outside is formed (Fig. 5). Subsequently, an inert gas is introduced at a flow rate which is set in advance from the operation space side gas supply unit 17, and thus, the inside of the operation space 10 becomes an atmosphere of the inert gas.

Since the flow rate of each gas is regulated such that the differential pressure occurring when the gas (buffer gas) taken in from the intake port 12 passes through the filter 121 becomes larger than the differential pressure occurring when the inert gas (operation space gas) in the operation space 10 passes through the filter 182 of the communication port 181, the operation space 10 is maintained in a negative pressure state with respect to the operation room 3.

Thereafter, an operator performs a necessary internal operation. This embodiment is the same as the first example in that at the time, even in a case where the internal operation involves a change in the internal volume of the operation space 10, the exhaust flow rate of the gas from the exhaust port 13 is set to an exhaust flow rate which is obtained by adding an exhaust flow rate, which is equivalent to the change speed of the internal volume when the internal volume decreases, to the already described exhaust flow rate for maintaining a negative pressure in the operation space 10 (including the exhaust flow rate which corresponds to the supply flow rate of the inert gas); and thereby, it is possible to maintain a negative pressure state in the operation space 10.

Subsequently, as illustrated in Fig. 6, it can be considered that as the operation portion such as the glove 14 falls off, the damage opening 110 is formed. In this case, when the differential pressure measurement unit 163 detects a decrease in the differential pressure between the operation room 3 and the operation space 10, and the differential pressure reaches a negative pressure with a magnitude set in advance, an operation is performed to close the supply stop mechanism 161 which is provided close to the intake port 12.

As a result, the taking in of the buffer gas from the intake port 12 is stopped, and the gas which flows into the GB 11 is only the gas which flows into the operation space 10 through the damage opening 110. Subsequently, since the pressure in the operation space 10 and the buffer space 10a increases, in a case where any regulation is not performed, the exhaust flow rate increases so that the pressure loss of the filter 121 which disappears by the formation of the damage opening 110 can be compensated by the filter 131, the exhaust pipe 21, or the flow rate regulation mechanism 22.

However, in the isolator system of this example, the flow rate regulation mechanism 22 prevents an increase in the exhaust flow rate which is involved by a pressure increase in the operation space 10, and performs the regulation of the flow rate in order to maintain the exhaust flow rate constant.

As a result, the flow speed of the gas which flows in through the damage opening 110 can be maintained at the predetermined lowest air speed (for example, 0.5 m/s), and the outflow of a substance which is being handled in the operation space 10 can be prevented.

Then, also in the GB 11 of this example, since the exhaust flow rate from the exhaust port 13 is maintained constant before and after the damage opening 110 is formed, a large pressure change does not occur in the operation room 3.

As described above, in the GB 11 of this example, since the inside of the operation space 10 is maintained at a negative pressure by introducing the buffer gas into the buffer space 10a, it is preferable to select the filter 182 with as small pressure loss as possible which is provided in the communication port 181 between the operation space 10 and the buffer space 10a. From this viewpoint, it is not indispensable to provide the filter 182 in the communication port 181. In a case where the substance which is being handled in the operation space 10 does not need to be prevented from entering the buffer space 10a, only the communication port 181 may be provided. However, such a configuration is not in accordance with the invention.

In addition, it is not indispensable to provide the operation space side gas supply unit 17 and supply the operation space gas. The configuration where the gas is not introduced into the operation space 10 may be adopted. Again, this configuration does not fall within the scope of the invention.

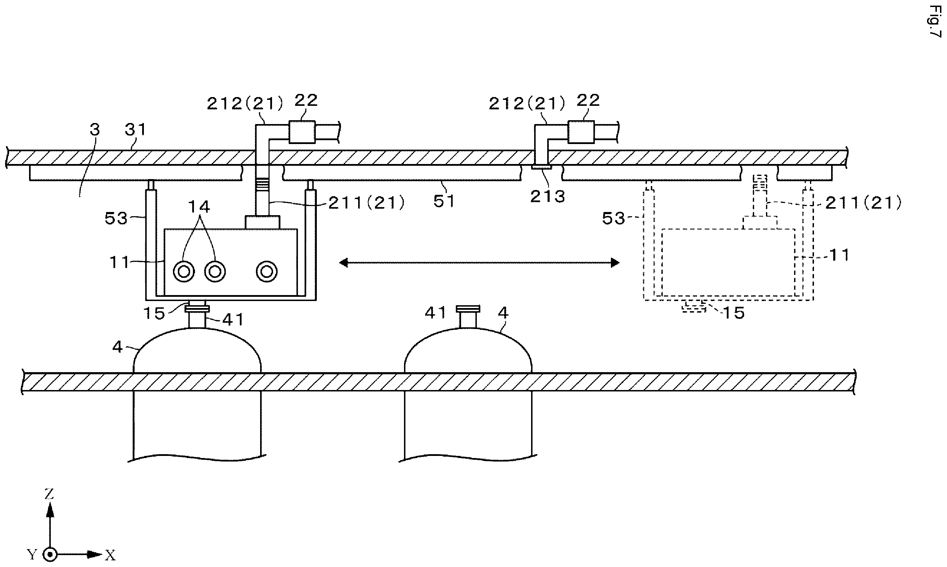

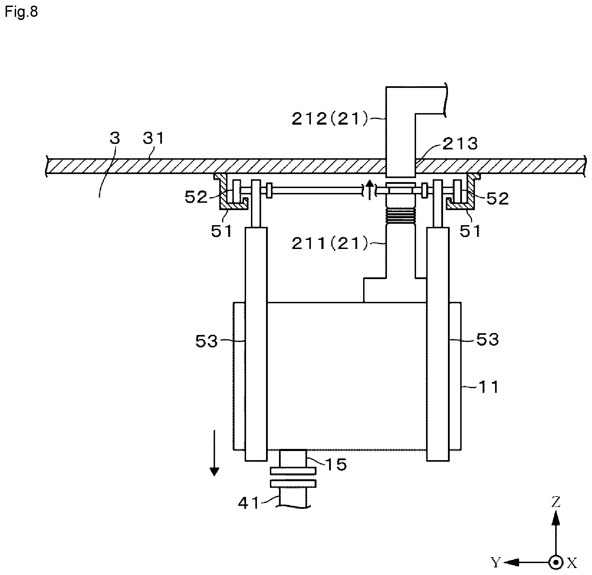

Subsequently, an example of an isolator system where the GB 11 is configured to be movable between a plurality of the reaction vessels 4 will be described with reference to Figs. 7 and 8.

In the example illustrated in Fig. 7, the plurality of reaction vessels 4 are arranged side by side in a state where an upper portion side of each of the reaction vessels 4 including the receiving pipes 41 described above is inserted into the operation room 3.

For example, a pair of two travel paths 51 having a rail shape are arranged on the ceiling portion 31 of the operation room 3, which is positioned above the reaction vessels 4, to extend along an arrangement direction of the reaction vessels 4. As illustrated in Fig. 8, a traveling body 52 including, for example, wheels and an axle is movably arranged on the travel paths 51. Suspension support portions 53 for supporting the GB 11 in a suspended manner are attached to the axle of the traveling body 52.

With the above-described configuration, the GB 11 is capable of freely moving in the operation room 3 along the travel paths 51 (Fig. 7).

In addition, as illustrated in Fig. 8, the exhaust pipe 21 which exhausts the gas from the GB 11 is divided into an indoor side exhaust pipe portion 211 attached to the GB 11, and an outdoor side exhaust pipe portion 212 which is arranged in a fixed manner in a state where the outdoor side exhaust pipe portion 212 extends on an upper surface side of the ceiling portion 31.

As a result, together with the GB 11 which is supported on the suspension support portions 53, the indoor side exhaust pipe portion 211 of the GB 11 is capable of moving in the operation room 3.

On the other hand, as illustrated in Fig. 7, a plurality of the outdoor side exhaust pipe portions 212 are arranged in a fixed manner on a ceiling portion side of the operation room 3 to each correspond to the arrangement position of each of the reaction vessels 4. Then, the flow rate regulation mechanism 22, the flow meter 221, or the controller 222 which is already described is provided in each of the outdoor side exhaust pipe portions 212 (only the flow rate regulation mechanism 22 is illustrated in Fig. 7).

Furthermore, for each of the outdoor side exhaust pipe portions 212, a connection port portion 213 is arranged on the ceiling portion of the operation room 3, and a distal end portion of the indoor side exhaust pipe portion 211 is detachable to the connection port portion 213.

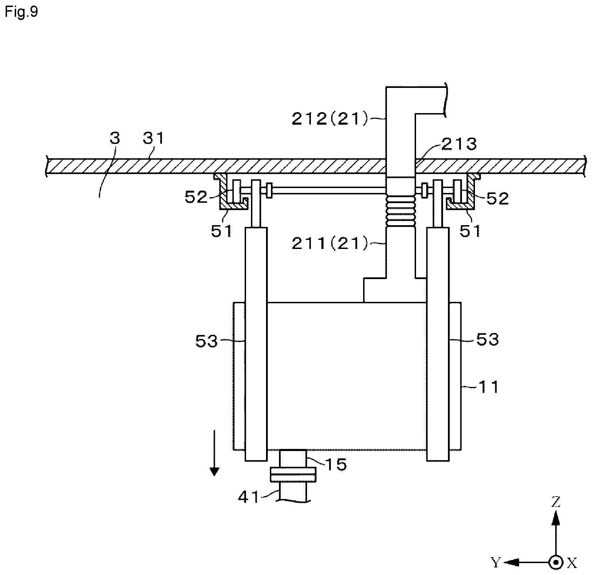

With the above-described configuration, the GB 11 moves toward the reaction vessel 4 which is a target where an internal operation is performed (Fig. 8), the input pipe 15 is connected to the reaction vessel 4, and a distal end portion of the indoor side exhaust pipe portion 211 is connected to the outdoor side exhaust pipe portion 212 (Figs. 7 and 9); and thereby, it is possible to form the isolator system which is described with reference to Figs. 1 and 2, Figs. 5 and 6, or the like.

At the time, since the plurality of outdoor side exhaust pipe portions 212 including the flow rate regulation mechanisms 22 are arranged in a fixed manner on the operation room 3 to correspond to the plurality of reaction vessels 4, it is not necessary to install a control mechanism of the flow rate regulation mechanism 22 which is configured to be movable together with the GB 11, and the like, thereby contributing to the weight reduction and the cost saving of the GB 11.

In addition, since, for example, the exhaust mechanism which is provided downstream of the outdoor side exhaust pipe portions 212 is common thereto, it is not necessary to provide a plurality of electric motors such as the exhaust fans, and it is not necessary to switch between the electric motors, which is a merit.

### Reference Signs List

- 10: Operation space
- 10a: Buffer space
- 11: Glove box (GB)
- 110: Damage opening
- 12: Intake port
- 121: Filter
- 122: Differential pressure regulation valve
- 13: Exhaust port
- 14: Glove
- 22: Flow rate regulation mechanism
- 3: Operation room
- 4: Reaction vessel
- 41: Receiving pipe

## Claims

1. An isolator system comprising:
a glove box (11) that includes an operation portion including a glove (14) for performing an internal operation, and forms an operation space (10) which is isolated from outside;
an intake port (12) for taking a gas into the glove box (11);
an exhaust port (13) connected to an exhaust pipe (21) for exhausting the gas in the glove box (11);
a flow rate regulation mechanism (22) that is configured to regulate an exhaust flow rate of the gas, which is exhausted from the exhaust port (13) to the exhaust pipe (21), to an exhaust flow rate where a differential pressure which is set in advance can be formed between the operation space (10) and the outside of the glove box (11) by a differential pressure forming portion (121) provided in the intake port (12),
**characterized by**
a partition member (18) which partitions an inside of the glove box (11) into the operation space (10) and a buffer space (10a) where the exhaust port (13) is formed, and in which a communication port (181) is formed to communicate the operation space (10) and the buffer space (10a) with each other;
a buffer side gas supply unit (16) that includes the intake port (12) and is configured to supply a buffer gas to the buffer space (10a);
an operation space side gas supply unit (17) that is configured to supply an operation space gas to the operation space (10); and
a filter (182) provided in the communication port (181),
wherein the buffer side gas supply unit (16) is configured such that the buffer gas is atmospheric air and the operation space side gas supply unit (17) is configured such that the operation space gas is an inert gas, and
wherein the operation space side gas supply unit (17) is configured such that a flow rate of the operation space gas supplied from the operation space side gas supply unit (17) is set at a flow rate where a pressure loss occurring when the buffer gas passes through a differential pressure forming portion (121) of the buffer side gas supply unit (16) becomes larger than a pressure loss occurring when the operation space gas passes through the filter (182).

2. The isolator system according to claim 1 further comprising:
a travel path (51) that extends along an arrangement direction of a plurality of reaction vessels (4) and is arranged above the reaction vessels (4) in an operation room where the reaction vessels (4) connectable to the glove box (11) are arranged;
a traveling body (52) that is provided to be movable along the travel path (51) in a state where the traveling body (52) is supported on the travel path (51); and
a suspension support portion (53) for supporting the glove box (11) in a suspended manner from the traveling body (52),
wherein the exhaust pipe (21) is connected to the exhaust port (13), and the exhaust pipe (21) includes an indoor side exhaust pipe portion (211) that moves along the travel path (51) as the glove box (11) moves, and outdoor side exhaust pipe portions (212) that are provided at a plurality of locations to correspond to the plurality of reaction vessels (4), and extend on an upper surface side of a ceiling portion of the operation room from connection port portions (213) which are provided in the ceiling portion and are configured to be detachable from a distal end portion of the indoor side exhaust pipe portion in a state where the glove box (11) is connected to the reaction vessel (4).

## Patentansprüche

1. Isolatorsystem, das aufweist:
einen Handschuhkasten (11), der einen Operationsabschnitt mit einem Handschuh (14) zur Durchführung einer internen Operation beinhaltet und einen Operationsraum (10) bildet, der von der Außenseite isoliert ist;
eine Ansaugöffnung (12) zum Aufnehmen eines Gases in den Handschuhkasten (11);
eine Ausstoßöffnung (13), die mit einem Ausstoßrohr (21) verbunden ist, um das Gas im Handschuhkasten (11) auszustoßen;
einen Strömungsratenregulierungsmechanismus (22), der so gestaltet ist, dass er eine Ausstoßströmungsrate des Gases, das aus der Ausstoßöffnung (13) zu dem Ausstoßrohr (21) ausgestoßen wird, auf eine Ausstoßströmungsrate reguliert, bei der ein Differenzdruck, der im Voraus eingestellt wird, zwischen dem Operationsraum (10) und der Außenseite des Handschuhkastens (11) durch einen Differenzdruckbildungsabschnitt (121) gebildet werden kann, der in der Ansaugöffnung (12) vorgesehen ist,
**gekennzeichnet durch**
ein Unterteilungselement (18), das ein Inneres des Handschuhkastens (11) in den Operationsraum (10) und einen Pufferraum (10a) unterteilt, in dem die Ausstoßöffnung (13) gebildet ist, und in dem eine Verbindungsöffnung (181) gebildet ist, um den Operationsraum (10) und den Pufferraum (10a) miteinander zu verbinden;
eine pufferseitige Gaszuführeinheit (16), die die Ansaugöffnung (12) beinhaltet und gestaltet ist, um ein Puffergas dem Pufferraum (10a) zuzuführen;
eine operationsraumseitige Gaszuführeinheit (17), die so gestaltet ist, dass sie dem Operationsraum (10) ein Operationsraumgas zuführt; und
einen Filter (182), der in der Verbindungsöffnung (181) vorgesehen ist,
wobei die pufferseitige Gaszuführeinheit (16) so gestaltet ist, dass das Puffergas atmosphärische Luft ist, und die operationsraumseitige Gaszuführeinheit (17) so gestaltet ist, dass das Operationsraumgas ein Inertgas ist, und
wobei die operationsraumseitige Gaszuführeinheit (17) so gestaltet ist, dass eine Strömungsrate des von der operationsraumseitigen Gaszuführeinheit (17) zugeführten Operationsraumgases auf eine Strömungsrate eingestellt ist, bei der ein Druckverlust, der auftritt, wenn das Puffergas durch einen Differenzdruckbildungsabschnitt (121) der operationsraumseitigen Gaszuführeinheit (16) hindurchgeht, größer wird als ein Druckverlust, der auftritt, wenn das Operationsraumgas durch den Filter (182) hindurchgeht.

2. Isolatorsystem nach Anspruch 1, das ferner aufweist:
einen Fahrweg (51), der sich entlang einer Anordnungsrichtung einer Mehrzahl von Reaktionsgefäßen (4) erstreckt und oberhalb der Reaktionsgefäße (4) in einem Operationsraum angeordnet ist, in dem die mit dem Handschuhkasten (11) verbindbaren Reaktionsgefäße (4) angeordnet sind;
einen Fahrkörper (52), der vorgesehen ist, um entlang des Fahrwegs (51) in einem Zustand bewegbar zu sein, in dem der Fahrkörper (52) an dem Fahrweg (51) gestützt ist; und
einen Aufhängungsstützabschnitt (53) zum Stützen des Handschuhkastens (11) in einer aufgehängten Weise von dem Fahrkörper (52),
wobei das Ausstoßrohr (21) mit der Ausstoßöffnung (13) verbunden ist und das Ausstoßrohr (21) einen innenseitigen Ausstoßrohrabschnitt (211), der sich entlang des Fahrwegs (51) bewegt, wenn sich der Handschuhkasten (11) bewegt, und außenseitige Ausstoßrohrabschnitte (212) beinhaltet, die an einer Mehrzahl von Stellen vorgesehen sind, um mit der Mehrzahl von Reaktionsgefäßen (4) übereinzustimmen, und sich auf einer oberen Flächenseite eines Deckenabschnitts des Operationsraums von Verbindungsöffnungsabschnitten (213) erstrecken, die in dem Deckenabschnitt vorgesehen und so gestaltet sind, dass sie von einem distalen Endabschnitt des innenseitigen Ausstoßrohrabschnitts in einem Zustand lösbar sind, in dem der Handschuhkasten (11) mit dem Reaktionsgefäß (4) verbunden ist.

## Revendications

1. Système isolateur, comprenant :
une boite à gant (11) qui inclut une partie d'opération incluant un gant (14) pour réaliser une opération interne, et forme un espace d'opération (10) qui est isolé de l'extérieur ;
un orifice d'admission (12) pour admettre un gaz dans la boîte à gant (11) ;
un orifice d'évacuation (13) raccordé à un tuyau d'évacuation (21) pour évacuer le gaz dans la boîte à gant (11) ;
un mécanisme de régulation de débit (22) qui est configuré pour réguler un débit d'évacuation du gaz, qui est évacué de l'orifice d'évacuation (13) au tuyau d'évacuation (21), à un débit d'évacuation où une pression différentielle qui est réglée d'avance peut être formée entre l'espace d'opération (10) et l'extérieur de la boite à gant (11) par une partie de formation de pression différentielle (121) prévue dans l'orifice d'admission (12),
**caractérisé par**
un élément de séparation (18) qui sépare un intérieur de la boite à gant (11) en l'espace d'opération (10) et un espace tampon (10a) où l'orifice d'évacuation (13) est formé, et dans lequel un orifice de communication (181) est formé pour faire communiquer l'espace d'opération (10) et l'espace tampon (10a) l'un avec l'autre ;
une unité d'alimentation en gaz côté tampon (16) qui inclut l'orifice d'admission (12) et est configurée pour effectuer l'alimentation en un gaz tampon à l'espace tampon (10a) ;
une unité d'alimentation en gaz côté espace d'opération (17) qui est configurée pour effectuer l'alimentation en un gaz d'espace d'opération à l'espace d'opération (10) ; et
un filtre (182) prévu dans l'orifice de communication (181),
dans lequel l'unité d'alimentation en gaz côté tampon (16) est configurée de telle sorte que le gaz tampon soit de l'air atmosphérique et l'unité d'alimentation en gaz côté espace d'opération (17) est configurée de telle sorte que le gaz d'espace d'opération soit un gaz inerte, et
dans lequel l'unité d'alimentation en gaz côté espace d'opération (17) est configurée de telle sorte qu'un débit du gaz d'espace d'opération, dont l'alimentation est effectuée à partir de l'unité d'alimentation en gaz côté espace d'opération (17), soit réglé à un débit où une perte de pression se produisant lorsque le gaz tampon passe à travers une partie de formation de pression différentielle (121) de l'unité d'alimentation en gaz côté tampon (16) devient supérieure à une perte de pression se produisant lorsque le gaz d'espace d'opération passe à travers le filtre (182).

2. Système isolateur selon la revendication 1, comprenant en outre :
un chemin de déplacement (51) qui s'étend le long d'une direction d'agencement d'une pluralité de récipients à réaction (4) et est agencé au-dessus des récipients à réaction (4) dans une salle d'opération où les récipients à réaction (4) raccordables à la boîte à gant (11) sont agencés ;
un corps se déplaçant (52) qui est prévu pour être mobile le long du chemin de déplacement (51) dans un état où le corps se déplaçant (52) est supporté sur le chemin de déplacement (51) ; et
une partie support à suspension (53) pour supporter la boite à gant (11) de manière suspendue à partir du corps se déplaçant (52),
dans lequel le tuyau d'évacuation (21) est raccordé à l'orifice d'évacuation (13), et le tuyau d'évacuation (21) inclut une partie tuyau d'évacuation côté intérieur (211) qui se meut le long du chemin de déplacement (51) lorsque la boite à gant (11) se meut, et des parties de tuyau d'évacuation côté extérieur (212) qui sont prévues à une pluralité d'emplacements pour correspondre à la pluralité de récipients à réaction (4), et s'étendent, sur un côté surface supérieure d'une partie plafond de la salle d'opération, depuis des parties orifices de raccordement (213) qui sont prévues dans la partie plafond et sont configurées pour être séparables d'une partie extrémité distale de la partie tuyau d'évacuation côté intérieur dans un état où la boîte à gant (11) est raccordée au récipient à réaction (4).
